# EUROPÄISCHE PATENTSCHRIFT

(11) **EP 1 663 270 B1**
(45) Veröffentlichungstag und Bekanntmachung des Hinweises auf die Patenterteilung: **20.08.2008**
(21) Anmeldenummer: 04765025.4
(22) Anmeldetag: 10.09.2004
(51) Int. Cl.: A61K 36/25, A61P 11/00

(54) **VERFAHREN ZUR HERSTELLUNG EINES EXTRAKTS AUS EFEUBLÄTTERN UND NACH DIESEM VERFAHREN HERGESTELLTER EXTRAKT**
METHOD FOR THE PRODUCTION OF IVY LEAF EXTRACTS, AND EXTRACT PRODUCED ACCORDING TO SAID METHOD
PROCEDE DE PRODUCTION D'UN EXTRAIT DE FEUILLES DE LIERRE, ET EXTRAIT OBTENU SELON CE PROCEDE

(30) Priorität: 19.09.2003 DE 10345343
(43) Veröffentlichungstag der Anmeldung: 07.06.2006
(73) Patentinhaber: Engelhard Arzneimittel GmbH & Co. KG, 61138 Niederdorfelden (DE)
(72) Erfinder: RUNKEL, Frank, 35418 Buseck (DE); SCHNEIDER, Wolfgang, 35510 Butzbach (DE); SCHMIDT, Oliver, 35418 Buseck (DE); ENGELHARD, Georg, Maximilian, 61476 Kronberg (DE)
(74) Vertreter: Weller, Wolfgang
(86) Internationale Anmeldenummer: PCT/EP2004/010092
(87) Internationale Veröffentlichungsnummer: WO 2005/037298

(56) Entgegenhaltungen:
- WO-A-20/04087183
- GB-A- 2 051 575
- DATABASE BIOSIS [Online] BIOSCIENCES INFORMATION SERVICE, PHILADELPHIA, PA, US; 1997, TRUTE ANDREAS ET AL: "In vitro antispasmodic compounds of the dry extract obtained from Hedera helix" XP009041755 Database accession no. PREV199799569819 & PLANTA MEDICA, Bd. 63, Nr. 2, 1997, Seiten 125-129, ISSN: 0032-0943
- DATABASE EMBASE [Online] ELSEVIER SCIENCE PUBLISHERS, AMSTERDAM, NL; 1986, WAGNER H ET AL: "Extracts of Hedera helix leaves: HPLC analysis" XP001204524 Database accession no. EMB-1987018570 & DEUTSCHE APOTHEKER ZEITUNG 1986 GERMANY, Bd. 126, Nr. 48, 1986, Seiten 2613-2617,

## Beschreibung

Die vorliegende Erfindung betrifft ein Verfahren zur Herstellung eines Extrakts aus Efeublättern sowie einen nach diesem Verfahren hergestellten Extrakt.

Extrakte aus Efeublättern werden heutzutage erfolgreich vor allem zur Therapie von Atemwegserkrankungen eingesetzt, da der Extrakt spasmolytische, expektorierende und antiobstruktive Wirkungen zeigt. Diese Wirkungen sind insbesondere auf die therapeutisch wichtigen Inhaltsstoffe der Efeublätterextrakte zurückzuführen, die der Klasse der Triterpensaponine angehören.

Hauptsaponin ist dabei das bisdesmosidische Hederacosid C und das daraus durch Esterhydrolyse entstehende α-Hederin. Als weiteres Saponin konnte Hederagenin nachgewiesen werden.

Da Extrakte aus Efeublättern durch verschiedene Verfahren gewonnen werden können, weisen diese Extrakte oftmals unterschiedliche Wirkungsgrade auf. Dies rührt daher, dass der Anteil der Inhaltsstoffe nicht nur von der natürlichen Zusammensetzung abhängt, sondern auch von der jeweiligen Methode der Extraktherstellung.

In neueren Studien der Anmelderin konnte gezeigt werden, dass es sich bei α-Hederin um die eigentlich wirksame Substanz der Efeublätter handelt und zur Bronchospasmolyse beiträgt, da diese Substanz über Bindung an β-adrenerge Rezeptoren und dadurch ausgelöste Kaskaden die glatte Muskulatur zur Erschlaffung bringt.

Extrakte aus Pflanzenmaterialien - insbesondere Trockenextrakte - sowie Verfahren zur Herstellung solcher Extrakte sind im Gebiet der Pharmazie und Arzneimittelzubereitungen vielfältig beschrieben.

Ein Verfahren zur Herstellung von Trockenextrakten aus Pflanzenmaterialien ist beispielsweise aus der DE 101 12 168 A1 bekannt. Mit dem dort offenbarten Verfahren soll der Gehalt an lipophilen und hydrophilen Substanzen eingestellt werden können. Hierbei wird das Pflanzenmaterial mindestens zwei Extraktionen mit Lösungsmitteln unterschiedlicher Lipophilie unterzogen und die Extrakte daraus separat gewonnen. Die Extrakte werden getrennt voneinander getrocknet und im gewünschten Verhältnis gemischt. Auf diese Weise kann der Gehalt an lipophilen und hydrophilen Substanzen eingestellt werden. Das Verfahren soll auch zur Gewinnung von Trockenextrakten aus Efeu (*Hedera helix*) geeignet sein.

In dieser Druckschrift ist jedoch weder ein Extrakt offenbart, der einen gezielt eingestellten Gehalt an α-Hederin und/oder Hederacosid C aufweist, noch ein Verfahren, mit welchem ein solcher Extrakt bereitgestellt werden könnte.

Ferner ist aus der DE 30 25 223 A1 eine pharmazeutische Zubereitung auf der Grundlage von Efeuextrakten und ein Verfahren zu deren Herstellung bekannt, wobei die Zubereitungen als Wirkstoff einen 90 % oder 60 % Hederasaponin C enthaltenden Extrakt oder α-Hederin aufweisen. Der Extrakt wird unter Verwendung von Aceton und Methanol gewonnen. Um Hederasaponin C, bzw. Hederacosid C, in α-Hederin umzuwandeln, und um dadurch einen Extrakt mit lediglich α-Hederin zu gewinnen, wird in der genannten Anmeldung der 90%-ige Extrakt mit Natriumhydroxid oder Kaliumhydroxid verseift.

Ein Verfahren, mit welchem ein Extrakt hergestellt werden kann, bei welchem α-Hederin und Hederacosid C mit einem beliebig eingestellten Gehalt vorliegen, ist jedoch auch in dieser Druckschrift nicht offenbart.

Damit eine reproduzierbare therapeutische Wirkung gewährleistet ist, sind aber gerade Efeuextrakte wünschenswert, die einen bestimmbaren Gehalt an Wirkstoffen aufweisen.

In DATABASE BIOSIS, BIOSCIENCES INFORMATION SERVICE, PHILA-DELPHIA, PA, US; 1997, TRUTE ANDREAS ET AL.: "In vitro antispasmodic compounds of the dry extract obtained from Hedera helix" XP009041755 Database accession no. PREV199799569819, sind in einer Tabelle unterschiedliche Extrakte beschrieben, die aus Hedera helix gewonnen werden. Diese Extrakte zeigen unterschiedliche Gehalte an Hederacosid C und α-Hederin, nämlich 11,0 und 1,2; 8,2 und 2,2; 9,1 und 1,5; 9,0 und 2,5; 5,0 und 2,3; sowie 6,2 und 1,4.

In DATABASE EMBASE, ELSEVIER SCIENCE PUBLISHERS, AMSTERDAM, NL; 1986, WAGNER H. ET AL.: "Extracts of Hedera helix leaves: HPLC analysis" XP001204524 Database accession no. EMB-1987018570 sind eine Vielzahl von Messdaten von Analysen unterschiedlicher Extrakte von Hedera-Proben beschrieben, wobei folgende prozentuale Gehalte von Hederacosid C und α-Hederin beschrieben sind, nämlich 20,18 und 1,27; 24,39 und 1,38; 15,30 und 0,83; 18,30 und 1,38; 4,07 und 2,13; 6,91 und 1,09; 4,52 und 1,54; 7,19 und 1,07; 4,39 und 8,48; 10,40 und 1,55; 4,65 und 1,68; sowie 6,01 und 0,40.

Aus der WO 2204/087183 A, die als Stand der Technik nach Art. 54 (3) EPÜ zu berücksichtigen ist, ist ein Extrakt offenbart, der 8,38 % α-Hederin und 5,88 % Hederacosid C enthält.

Nachteilig an den bisher eingesetzten Extrakten ist ferner, dass diese Extrakte mehrfach appliziert werden müssen, um eine durch α-Hederin auszulösende sofortige und dauerhafte Bronchospasmolyse zu bewirken. Die Ursache hierfür kann in der ungenügenden Bioverfügbarkeit von α-Hederin liegen, da beispielsweise der in den Extrakten vorliegende α-Hederin-Gehalt bereits zu Beginn der Einnahme gering ist. Andererseits wird auch ursprünglich im Extrakt vorhandenes α-Hederin *in vivo* relativ schnell resorbiert, wodurch Mehrfachapplikationen notwendig werden, um eine therapeutische Wirkung zu erzielen.

Unter Berücksichtigung der obigen Ausführungen ist es daher Aufgabe der vorliegenden Erfindung, ein Verfahren zur Herstellung eines Extrakts sowie einen Extrakt bereitzustellen, durch den eine rasche Bioverfügbarkeit von α-Hederin und die Aufrechterhaltung der Bioverfügbarkeit über einen längeren Zeitraum hinweg gewährleistet ist.

Die der Erfindung zu Grunde liegende Aufgabe wird durch ein Verfahren zur Herstellung eines Extraktes aus Efeublättern mit einem einstellbaren Gehalt an Hederacosid C und α-Hederin gelöst, wobei das Verfahren die folgenden Schritte aufweist:
a) Bereitstellen eines ersten α-Hederin-reichen Extraktes, der mindestens 3 % α-Hederin aufweist;
b) Bereitstellen eines zweiten Hederacosid C-reichen Extraktes, der mindestens 5 % Hederacosid C aufweist; und
c) Vermengen des ersten und des zweiten Extraktes zu einem Extrakt, der einen eingestellten Hederacosid C-Gehalt und einen eingestellten α-Hederin-Gehalt aufweist.

Mit dem erfindungsgemäßen Verfahren kann ein Extrakt gewonnen werden, welcher zum einen α-Hederin als sofort wirkende Substanz vorliegen hat und andererseits auch Hederacosid c, welches zu α-Hederin *in vivo* umgewandelt wird. Dabei weist der erste Extrakt α-Hederin und der zweite Extrakt Hederacosid C jeweils in stark angereicherter Form auf.

Der erste Extrakt kann dabei bspw. dadurch hergestellt werden, dass das Ausgangsmaterial, bspw. die getrocknete Droge, zunächst zerkleinert, anschließend einem Fermentationsschritt unterzogen und danach bspw. in einem Alkohol/Wasser-Gemisch, bspw. Ethanol 30 %, vorgequollen und extrahiert wird. Abschließend kann bspw. eine Dünnfilmvaporisation und eine Sprühtrocknung erfolgen. Je nach erwünschtem α-Hederin-Gehalt kann die ganze Droge fermentiert werden - auf diese weise wird alles in der Ausgangsdroge vorliegende Hederacosid C in α-Hederin umgewandelt. Andererseits kann auch nur ein Teil der Ausgangsdroge fermentiert werden und die restlichen Teile erst zur Vorquellung in Ethanol beigemengt werden - dadurch liegt α-Hederin lediglich stark angereichert vor.

"Fermentieren" bedeutet vorliegend der Abbau bzw. die Umwandlung von in einer Ursubstanz vorliegenden Inhaltsstoffen in andere Stoffe unter Zugabe eines Fermentations-Mediums, bspw. Wasser, zu der Ursubstanz, wobei ggf. bestimmte Parameter, bspw. Zeit und Temperatur, auf den Fermentations-Prozess abgestimmt werden. Dieser neue Fermentationsschritt eröffnet die Möglichkeit, gezielt α-Hederin-reiche Extrakte herzustellen.

Die Efeublätter können dabei bspw. als getrocknete Droge eingesetzt werden. Getrocknete Drogen haben in der Arzneimittelherstellung den Vorteil, dass sie bezüglich der Haltbarkeit u.U. besser zu handhaben sind als frische Drogen. Dennoch ist die Verwendung von frischen Efeublättern bei dem erfindungsgemäßen Extrakt nicht ausgeschlossen.

Getrocknete Arzneipflanzen und Arzneipflanzenteile werden im Gebiet der Arzneimitteltechnik definitionsgemäß als "Drogen" bezeichnet. Die Verwendung von solchen als "Drogen" vorliegenden Arzneipflanzen kann dabei entweder in unveränderter oder in zerkleinerter Form erfolgen.

Der zweite Extrakt kann bspw. dadurch hergestellt werden, dass die getrocknete Droge direkt nach dem Schneiden mit einem Extraktionsmittel, bspw. Ethanol 30 %, versetzt und nach gängigen Methoden extrahiert wird. Alternativ kann die zerkleinerte Droge - vor der Extraktion mit Ethanol - mit heißem Wasserdampf bedampft werden. Versuche der Erfinder haben gezeigt, dass durch diese Behandlung der Gehalt an Hederacosid C im Extrakt erhöht, bzw. bezüglich des Ausgangsgehalts stabilisiert, und der α-Hederin-Gehalt gesenkt werden konnte. Auf diese Weisen werden Extrakte gewonnen, die in dem erfindungsgemäßen Verfahren in Schritt b) als zweiter, Hederacosid C-reicher Extrakt eingesetzt werden können. Dieser neue Bedampfungsschritt eröffnet die Möglichkeit, gezielt Hederacosid C-reiche Extrakte herzustellen.

Bei dem Verfahren weist der α-Hederin-reiche Extrakt in Schritt a) einen α-Hederin-Gehalt von mindestens 3 %, insbesondere von 5 % auf.

Der Hederacosid C-reiche Extrakt in Schritt b) weist einen Hederacosid C-Gehalt von mindestens 5 %, und insbesondere einen Hederacosid C-Gehalt von mindestens 10 % und einen α-Hederin-Gehalt von weniger als 2 % auf.

Dabei ist bevorzugt, wenn bei dem Verfahren der in Schritt c) zu gewinnende Extrakt einen Hederacosid C-Gehalt von ca. 3 bis ca. 10 %, insbesondere von ca. 6,5 %, und einen α-Hederin-Gehalt von ca. 1 bis ca. 7 %, insbesondere von ca. 4,0 % aufweist.

Die der Erfindung zu Grunde liegende Aufgabe wird ferner gelöst durch einen Extrakt, der nach dem erfindungsgemäßen Verfahren hergestellt ist. Insbesondere wird die der Erfindung zu Grunde liegende Aufgabe gelöst durch einen nach dem erfindungsgemäßen Verfahren hergestellten Extrakt aus Efeublättern mit einem eingestellten Hederacosid C-Gehalt von ca. 3 % bis ca. 10 %, insbesondere von ca. 6,5 %, und einem eingestellten α-Hederin-Gehalt von ca. 1 % bis ca. 7 %, insbesondere von ca. 4,0 %.

Der erfindungsgemäße Extrakt bietet aufgrund des eingestellten Gehalts an den besagten Wirkstoffen nämlich den Vorteil, dass nach einer Anwendung des Extraktes der Wirkstoff α-Hederin zunächst durch das im Extrakt als solches vorliegende α-Hederin rasch verfügbar gemacht wird. Das in dem Extrakt neben α-Hederin vorliegende Hederacosid C wird daneben *in vivo* zeitverzögert resorbiert, bzw. nach und nach in α-Hederin umgewandelt, so dass nach Aufbrauchen des ursprünglich vorliegenden α-Hederins das aus Hederacosid C umgewandelte α-Hederin verfügbar ist. Dadurch wird vorteilhaft eine längere therapeutische Wirksamkeit des Extraktes, bzw. des den Extrakt aufweisenden Arzneimittels, gewährleistet.

Die Erfinder haben diesen Prozess in eigenen Versuchen nachgewiesen. In diesem Zusammenhang zeigten sie, dass mit einem erfindungsgemäßen Extrakt, bei dem der Wirkstoff-Gehalt von α-Hederin und Hederacosid C in dem o.a. optimalen Bereich eingestellt war, ein rasches Anfluten und der Erhalt einer konstanten Konzentration erzielt werden konnte. Ein Vergleichsextrakt, der einen höheren Hederacosid C-Gehalt und einen niedrigeren α-Hederin-Gehalt aufwies, musste im Vergleich zu dem erfindungsgemäßen Extrakt öfters appliziert werden, um vergleichbare Konzentrationswerte zu erzielen. Demnach ist es mit dem neuen Extrakt möglich, bereits durch eine einmalige Applikation eine α-Hederin-Konzentration zu bewirken, die für eine Anwendung zur Bronchospasmolyse wünschenswert ist.

Dabei ist einbesondere bevorzugt, wenn der Extrakt nach dem erfindungsgemäßen Verfahren hergestellt ist und einen Hederacosid C-Gehalt von ca. 6,5 % und einen α-Hederin-Gehalt von ca. 4,0 % aufweist.

Die Erfindung betrifft ferner die Verwendung des erfindungsgemäßen Extraktes zur Herstellung eines Arzneimittels, insbesondere zur Behandlung von Atemwegserkrankungen, sowie ein Arzneimittel, welches den erfindungsgemäßen Extrakt aufweist.

Die erfindungsgemäßen Extrakte mit einem eingestellten Gehalt der Inhaltsstoffe Hederacosid C und α-Hederin sind in der Verwendung als Arzneimittel besonders vorteilhaft, da sich mit ihnen reproduzierbare therapeutische Wirkungen erzielen lassen und außerdem eine sofortige Versorgung mit dem spasmolytisch aktiven Wirkstoff α-Hederin ermöglicht wird, wobei die Versorgung über einen längeren Zeitraum konstant aufrecht erhalten wird.

Das erfindungsgemäße Arzneimittel kann danach zur Behandlung von Atemwegserkrankungen wie bspw. infektiös-entzündliche Atemwegserkrankungen eingesetzt werden, wie bspw. Pneumonie, Tracheitis, Bronchitis etc., aber auch bei obstruktiven und restriktiven Lungenerkrankungen wie chronische Bronchitis, Asthma bronchiale, Bronchiektasen usw., also bei Atemwegserkrankungen, bei welchen eine Relaxierung der schwachen Muskulatur erwünscht ist.

Das Arzneimittel kann in Form von Kapseln, Tabletten, Dragees, Zäpfchen, Granulat, Pulver, Lösungen, Cremes, Emulsionen, Aerosolen, Salben und Ölen vorliegen. Orale Verabreichungsformen sind dabei insbesondere bevorzugt. Das Arzneimittel kann dabei Hilfsstoffe aufweisen, die herkömmlich in der Arzneimittelherstellung verwendet werden. Eine Reihe von geeigneten Substanzen findet sich bspw. in A. Kibbe, Handbook of Pharmaceutical Excipients, 3. Ed. 2000, American Pharmaceutical Association and Pharmaceutical Press.

Weitere Vorteile ergeben sich aus dem Beispiel und der beigefügten Figur. Es zeigt:

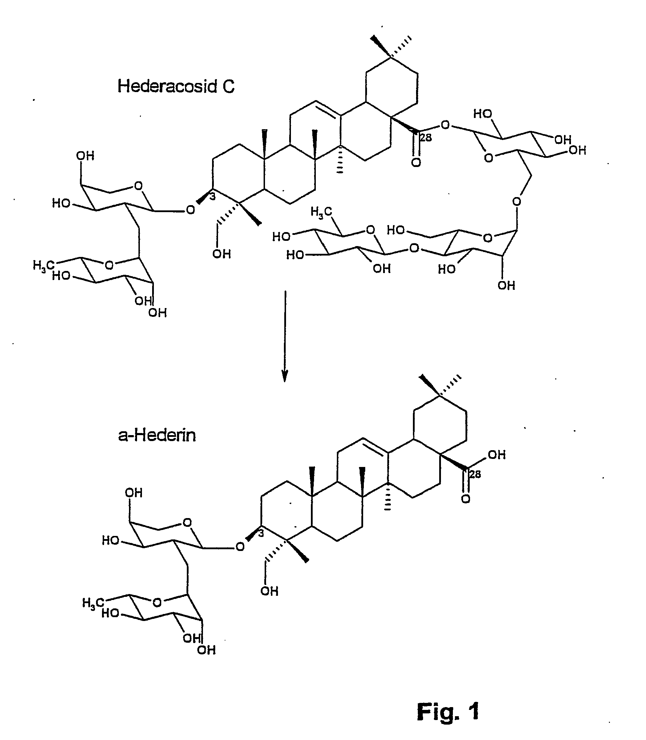

- Fig. 1: die Hydrolyse von Hederacosid C zu α-Hederin.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

### Beispiel

### 1. Resorptionsverhalten von Efeublätterinhaltsstoffen in vivo (Ratten)

Im Rahmen einer Resorptionsstudie mittels des Biopharmazeutischen Klassifizierungssystem (BCS) konnte gezeigt werden, dass α-Hederin eine mittlere Absorption (10-90%) beizumessen ist. Hederacosid C permeierte unter gleichen Bedingungen nicht durch die Membranen, weshalb eine mögliche Resorption von Hederacosid C in Form des α-Hederin untersucht werden sollte. Dazu wurden Resorptionsstudien an Ratten durchgeführt.

Im Folgenden wurde der Einfluss der Applikation zweier verschiedener Extrakte auf die Plasmakonzentration von α-Hederin untersucht. Ferner wurde die Veränderung der Plasmakonzentration von α-Hederin bei Einmalapplikation im Vergleich zur Mehrfachapplikation des Extraktes und bei verschiedenen Blutentnahmezeiten nach der Applikation untersucht, sowie die Resorptionsquote von α-Hederin. Hierzu wurden zwei Extrakte ausgewählt, deren Saponinverteilung in der folgenden Tabelle 1 wiedergegeben ist:

**Tabelle 1: Saponinverteilung der Extrakte**

| | Gehalt Hederacosid C [%] | Gehalt α-Hederin [%] |
|---|---|---|
| Extrakt A | 14,5 | 0,9 |
| Extrakt B | 6,5 | 4,0 |

Für die intravenöse Applikation wurde die benötigte Menge Extrakt in Ethanol 11 % (m/m) gelöst, abfiltriert und injiziert. Dabei wurde ein Applikationsvolumen von 2 ml Extraktlösung angestrebt (jeweilige Dosis [mg/kg Körpergewicht]: 25). 5 Minuten nach der Applikation wurde den Tieren eine Blutprobe entnommen. Pro Probe wurden etwa 2 ml Vollblut mit EDTA-Zusatz zur Gerinnungshemmung benötigt.

Zur peroralen Applikation wurden Suspensionen der Extrakte in Glycerol hergestellt. Die Tiere wurden mit ca. 2 ml dieser Suspensionen geschlundet (jeweilige Dosis [mg/kg Körpergewicht]: 1000 und 166,66). 1 und 3 Stunden nach der jeweils letzten Applikation wurde den Tieren jeweils 2 ml Blut entnommen. Die Tiere hatten ein durchschnittliches Gewicht von ca. 200 g und ein Blutvolumen von ca. 20 ml.

Die Ergebnisse der Analysen sind in der folgenden Tabelle 2 dargestellt:

**Tabelle 2: Übersicht Verabreichung/α-Hederin-Plasmakonzentration**

| Probenart | Applikation | Appl. Extrakt | Appl. Dosis [mg/kg KG] | Blutentnahme-Zeitpunkt | Plasmakonzentration α-Hederin in [µg/ml] |
|---|---|---|---|---|---|
| Blind-Probe | Keine | - | - | - | 0,0 |
| Referenz | i.v. | A | 25 | 5 min nach Appl. | 2,3 |
| Probe | p.o. Einmalappl. | A | 1000 | 1 h nach Appl. | 3,3 |
| Probe | p.o. Einmalappl. | A | 1000 | 3 h nach Appl. | 6,3 |
| Probe | p.o. 3 Tage 2x tägl. | A | 166,66 | 1 h nach letzter Appl. | 20,8 |
| Referenz | i.v. | B | 25 | 5 min. nach Appl. | 10,0 |
| Probe | p.o. Einmalappl. | B | 1000 | 1 h nach Appl. | 11,1 |
| Probe | p.o. Einmalappl. | B | 1000 | 3 h nach Appl. | 14,4 |
| Probe | p.o. 3 Tage 2x tägl. | B | 166,66 | 1 h nach letzter Appl. | 14,9 |

Hederacosid C konnte in keiner Probe detektiert werden-Wie der oben stehenden Tabelle 2 entnommen werden kann, ist bei einer Mehrfachapplikation eines α-Hederin-ärmeren Extraktes eine höhere α-Hederin-Plasmakonzentration gemessen worden als bei der vergleichenden Applikation eines an α-Hederin reicheren Extraktes. Dieses Ergebnis konnte durch eine Umrechnung erläutert werden, welche in der folgenden Tabelle 3 gezeigt ist:

**Tabelle 3: Resorptionsguoten für die Applikation des Extraktes A (14,5 % Hederacosid C und 0,9 % α-Hederin)**

| Applikationsart/ Blutabnahme | Plasmakonz. α-Hederin [µg/ml] | Max. erreichbare Konz. (theoretisch) | Resorptions-Quote [%] | Max. erreichbarer Blutspiegel bei Betrachtung des Gesamtsaponingehalts [µg/ml] | Resorptions-Quote [%] |
|---|---|---|---|---|---|
| Einfachappl./ 1 h nach Appl. | 3,3 | 90 | 3,7 | 980 | 0,33 |
| Einfachappl./ 3 h nach Appl. | 6,3 | 90 | 7,0 | 980 | 0,64 |
| Mehrfachappl./ 1 h nach Appl. | 20,8 | 90 | 23,1 | 980 | 2,12 |

Der Gesamtsaponingehalt ergibt sich unter Berücksichtigung der molaren Massen beider Inhaltsstoffe und ist auf α-Hederin bezogen. Beim hier vorliegenden Extrakt A betrug der Gesamtsaponingehalt 9,8 %, bezogen auf α-Hederin. Ein Blutvolumen von 20 ml wurde angenommen.

Die Berechnungen für Extrakt B sind in der nachfolgenden Tabelle 4 gezeigt:

**Tabelle 4: Resorptionsquoten für die Applikation des Extraktes B (6,5 % Hederacosid C und 4,0 % α-Hederin)**

| Applikationsart/ Blutabnahme | Plasmakonz. α-Hederin [µg/ml] | Max. erreichbare Konz. (theoretisch) | Resorptions-Quote [%] | Max. erreichbarer Blutspiegel bei Betrachtung des Gesamtsaponingehalts [µg/ml] | Resorptions-Quote [%] |
|---|---|---|---|---|---|
| Einfachappl./ 1 h nach Appl. | 11,1 | 400 | 2,8 | 800 | 1,38 |
| Einfachappl./ 3 h nach Appl. | 14,4 | 400 | 3,6 | 800 | 1,80 |
| Mehrfachappl./ 1 h nach Appl. | 14,9 | 400 | 3,7 | 800 | 1,86 |

Der Gesamtsaponingehalt ergibt sich unter Berücksichtigung der molaren Massen beider Inhaltsstoffe und ist auf α-Hederin bezogen. Beim hier vorliegenden Extrakt B beträgt der Gesamtsaponingehalt 9,8 %, bezogen auf α-Hederin. Ein Blutvolumen von 20 ml wurde angenommen.

Zusammenfassend lässt sich sagen, dass die Mehrfachapplikation eines α-Hederin-reichen und eines α-Hederin-armen Extraktes zu vergleichbaren Plasmakonzentrationen von α-Hederin führten. Berücksichtigt man jedoch den Hederacosid C-Gehalt beider Extrakte und bestimmt den Gesamtgehalt beider Saponine, berechnet als α-Hederin, so unterscheiden sich beide Extrakte nur geringfügig. Aufgrund dieser Ergebnisse muss davon ausgegangen werden, dass *in vivo* eine Abspaltung des C28-ständigen Zuckers des Hederacosid C stattfindet, die resorbierbares α-Hederin entstehen lässt. Dieser Prozess - also die Umwandlung von Hederacosid C in α-Hederin - ist in Fig. 1 gezeigt.

Mit dem *in vitro* Nachweis einer spasmolytischen Aktivität von α-Hederin im Gegensatz zu Hederacosid C und der Wiederfindung von α-Hederin im Plasma im Gegensatz zu Hederacosid C, kann davon ausgegangen werden, dass die α-Hederin-Konzentration im Blut maßgeblich mit der in vielen klinischen Studien belegten therapeutischen Wirkung korreliert.

Mit einem in Bezug auf die therapeutische Wirkung optimierten Extrakt sollte daher eine einheitliche Plasmakonzentration rasch erreicht werden, die auch bei Mehrfachapplikation des Extraktes aufrecht erhalten wird. Ein solcher Extrakt stellt der Extrakt B dar, dessen Applikation - im Gegensatz zu Extrakt A - schon nach einer Stunde zu einer Plasmakonzentration führt, die mit der Plasmakonzentration nach Mehrfachapplikation vergleichbar ist. Extrakt B mit einem Gehalt von 6,5 % Hederacosid C und 4,0 % α-Hederin zeichnete sich durch einen schnellen Wirkeintritt und durch eine Reproduzierbarkeit der therapeutischen Wirkung aus.

Der für die therapeutische Wirkung optimale Bereich bezüglich des Gehalts an Hederacosid C und α-Hederin in einem Extrakt kann in Anlehnung an Extrakt B daher wie folgt angegeben werden:
**Hederacosid C: 5 - 8 %**
**α-Hederin: 3 - 5 %**

### 2. Herstellung eines optimierten Efeu-Extrakts

Der optimierte Efeu-Extrakt zeichnet sich durch ein in gewissen Grenzen chargenkonformes Inhaltsspektrum - bezogen auf die Inhaltsstoffe Hederacosid C und α-Hederin - aus. Der Gehalt an Hederacosid C sollte dabei auf 50 bis 80 mg/g und der Gehalt an α-Hederin auf 30 bis 50 mg/g spezifiziert werden. Um diese Spezifikation einzuhalten, wurden mehrere Extrakte gemischt. Die Extrakte, die durch konventionelle Verfahren gewonnen werden, eignen sich jedoch nur bedingt für solche Mischprozesse, da sie oftmals keinen ausreichend hohen Gehalt am betreffenden Wirkstoff aufweisen.

Nachfolgend sind Extraktionsverfahren beschrieben, die zu Efeublätter-Extrakten führen, welche sich jeweils durch einen hohen Gehalt eines der beiden Hederasaponine auszeichnen, während gleichzeitig das jeweils andere Hederasaponin nur in sehr geringen Mengen enthalten ist.

### a) Herstellung eines α-Hederin-reichen Extraktes

Bei diesem Extraktionsverfahren ist es für die Steuerung des Verhältnisses von Hederacosid C zu α-Hederin Grundlage, die Abspaltung des C28-ständigen Zuckers des Hederacosid C gezielt in das Extraktionsverfahren einzubeziehen. Da eine Umwandlung von Hederacosid C in α-Hederin nahezu quantitativ abläuft, ist praktisch jede Blattcharge als Ausgangsmaterial zur Herstellung eines α-Hederin-reichen Extraktes geeignet.

Nach Prüfung der Qualität und Freigabe durch die Qualitätskontrolle wurde ein Teil der Droge (Efeublätter DAC) in einer Mühle stark zerkleinert, wobei eine Schutzsiebung eine Größe der Fragmente von maximal 2 x 2 mm garantierte. Zusätzlich wurde das gesiebte Gut optisch auf größere Partikel und Verunreinigungen geprüft.

Zu der zerkleinerten Probe wurde der Wasseranteil aus 6 Teilen Extraktionsmittel (Ethanol 30 % (m/m)) gegeben-Dieser Ansatz wurde bei 30 °C unter gelegentlichem Vermischen/Umrühren für 60 min fermentiert.

Anschließend wurde der Anteil Ethanol 96 % aus 6 Teilen des Extraktionsmittels zugegeben und der Ansatz durch Rühren homogenisiert.

Nach einer 6-stündigen Vorquellphase wurde das Eluat abgetrennt und die zurückbleibende Droge mit den restlichen 6 Teilen des Extraktionsmittels perkoliert.

Die vereinten Eluate wurden zum Ausschluss kleiner Drogenpartikel nochmals filtriert und homogenisiert, bevor sie mittels Dünnfilmvaporisation bei 55 °C und 150 mbar zum Spissum getrocknet wurden. Dieses wurde homogenisiert und anschließend per Sprühtrocknung bei 45 bis 60 °C zum Efeublättertrockenextrakt getrocknet.

Zur Überprüfung dieses Herstellungsverfahrens wurden folgende Extrakte hergestellt: Ausgehend von Efeublättern mit einem Gehalt von 3,91 % Hederacosid C und 0,20 % α-Hederin wurde einmal nach der konventionellen Methode (Zerkleinern der getrockneten Probe mit direkt anschließendem Versetzen mit 30% (m/m) Ethanol und Extraktion) und einmal nach der neuen Methode ein Extrakt angefertigt. In der nachfolgenden Tabelle 5 sind die chromatographischen Ergebnisse bezüglich des Gehaltes an α-Hederin und Hederacosid C wiedergegeben:

**Tabelle 5: Gehalt an α-Hederin und Hederacosid C in den Extrakten**

| | α-Hederin (%) | Hederacosid C (%) | Gesamtsaponine berechnet als Hederacosid C (%) |
|---|---|---|---|
| Konventionell | 0,53 | 8,68 | 9,54 |
| Neu | 4,74 | 0 | 7,71 |

Wie der Tabelle zu entnehmen ist, konnte mit dem neuen Extraktionsverfahren das in den Blättern enthaltene Hederacosid C vollständig in α-Hederin umgewandelt werden.

Da sich auch der Gesamtsaponingehalt, berechnet als Hederacosid C, in derselben Größenordnung bewegt, ist man - bei Kenntnis der entsprechenden Saponinkonzentration in der Droge und unter Berücksichtigung des Anreicherungsfaktors von ca. 2 bis 3 - in der Lage, den letztendlichen α-Hederin-Gehalt des Extraktes abschätzen.

Um nur einen Teil des in der Droge vorliegenden Hederacosid C in α-Hederin umzuwandeln, kann demnach nur ein bestimmter Anteil der Droge der Fermentation mit Wasser unterzogen werden, wobei alle anderen Parameter konstant bleiben. Nach Ablauf der 60-minütigen Fermentation wird dann für die 6-stündige Vorquellung die restliche Droge und das Ethanol zugesetzt. Bei Kenntnis der entsprechenden Saponinkonzentration in der Droge und unter Berücksichtigung des Anreicherungsfaktors von ca. 2-3 kann man den letztendlichen α-Hederin-Gehalt des Extraktes abschätzen.

Mit dem erfindungsgemäßen Verfahren ist es demnach möglich, das Inhaltsstoffspektrum eines Efeublättertrockenextraktes durch Einführung einer Fermentation deutlich zu ändern, ohne einen großen Zeit- und Ressourcenaufwand anzuwenden. Nicht zuletzt durch die zahlreichen Veröffentlichungen über die Wirksamkeit von α-Hederin wirkt sich die gezielte Beeinflussung des α-Hederin-Gehalts in einem Trockenextrakt, bzw. Arzneimittels, auf die Wirksamkeit des Extraktes positiv aus.

### b) Herstellung von Efeublättertrockenextrakten mit erhöhtem Hederacosid C-Gehalt

Grundlage für die Steuerung des Verhältnisses von Hederacosid C zu α-Hederin ist es, die Abspaltung des C28- ständigen Zuckers des Hederacosid C während des Extraktionsverfahrens gezielt zu unterbinden. Bei der Auswahl der Drogencharge sollte darauf geachtet werden, dass Blattchargen mit niedrigem α-Hederin-Gehalt eingesetzt werden. Eine Spezifizierung auf unter 0,5 % α-Hederin in Bezug auf die getrocknete Droge ist empfehlenswert.

Eine homogene Probe Efeublätter wurde bezüglich des Gehalts der beiden Saponine wie folgt analysiert:
**Hederacosid C: 6,37 %**
α**-Hederin: 0,85 %**

Ausgehend von der Droge wurden je drei Extraktionen nach folgender Extraktionsvorschrift durchgeführt:

3 g der getrockneten und auf ca. 3x3 mm zerkleinerten Droge wurden mit heißem Wasserdampf für einige Sekunden bedampft. Die so behandelte Droge wurde mit 18 g des Extraktionsmittels (Ethanol 30 % (m/m)) ca. 6 Stunden vorgequollen. Nach Ablassen der Miscella wurde die verbleibende Droge mit weiteren 18 g des Extraktionsmittels perkoliert. Diese Miscella wurde im Vakuumtrockenschrank getrocknet. Alternativ kann die Trocknung bspw. durch Dünnfilmvaporisation bei bspw. 55 °C und 150 mbar und anschließender Sprühtrocknung bei 45 - 60 °C erfolgen.

Die Extraktionstemperatur liegt bevorzugt zwischen ca. 20 und ca. 40 °C, insbesondere bei ca. 30 °C. Das Verhältnis von Droge zum Extraktionsmittel liegt dabei bspw. bei 1:12.

Nach Analyse der erhaltenen Extrakte konnten die in der folgenden Tabelle 6 aufgeführten Ergebnisse dokumentiert werden:

**Tabelle 6: Gehalt an Hederacosid C und α-Hederin in den bedampften Extrakten**

| Probe | Gehalt Hederacosid C | Gehalt α-Hederin | Gehalt Hederasaponine, berechnet als Hederacosid C |
|---|---|---|---|
| | [%] | [%] | [%] |
| Ausgangsdroge | 6,37 | 0,85 | 7,75 |
| Extrakt bedampft 1 | 14,33 | 0,85 | 15,71 |
| Extrakt bedampft 2 | 14,26 | 0,85 | 15,64 |
| Extrakt bedampft 3 | 14,57 | 0,71 | 15,72 |

Der nach dieser Vorschrift hergestellte Blättertrockenextrakt wies demnach die maximal in den eingesetzten Blättern enthaltene Menge an α-Hederin auf. Insgesamt wurden somit lagerstabile Extrakte mit hohem Hederacosid C-Gehalt und sehr niedrigem α-Hederin-Gehalt erhalten. Bei Verwendung von Blattchargen mit einem Gehalt von unter 0,5 % α-Hederin kann auch für den erhaltenen Extrakt ein Höchstgehalt von 0,5 % α-Hederin angenommen werden.

### c) Mischung der beiden angereicherten Extrakte

Mittels der unter 2a) und 2b) beschiebenden Extraktionsverfahren können Efeublätterextrakte hergestellt werden, die jeweils eines der beiden Hederasaponine in angereicherter Form enthalten.

Mit den beiden angereicherten Extrakten wird schließlich eine Mischung zum fertigen Spezialextrakt mit einem Gehalt von 5 - 8 % Hederaco s id C und 3 - 5 % α-Hederin hergestellt.

### Beispiel:

1 Teil Extrakt A mit ca. 7,5 % α-Hederin +
1 Teil Extrakt B mit ca. 13,0 % Hederacosid C und 0,5 % α-Hederin
-> Extrakt mit 6,5 % Hederacosid und 4,0 % α-Hederin.

Diese Rechnung ist natürlich jedes Mal den Gehaltswerten der beiden angereicherten Extrakten anzupassen, aber auch ein Einstellen der beiden angereicherten Extrakte auf die im Beispiel genannten Konzentrationen durch Mischung verschiedener angereicherter Extrakte ist denkbar.

Es ist nunmehr möglich, ein Arzneimittel zu formulieren, das schon bei einmaliger Verabreichung dafür sorgt, dass der eigentliche Wirkstoff (α-Hederin) sehr schnell im Körper zur Verfügung steht. Durch den gleichzeitig hohen Hederacosid C-Gehalt kann für lange Zeit ein hoher α-Hederin-Spiegel aufrecht erhalten werden, da im Körper das Hederacosid C laufend zu α-Hederin umgewandelt wird.

Dadurch muss das Arzneimittel nicht mehrfach eingenommen werden, was die so genannte Patienten-Compliance erhöht. Ein Anwendungsgebiet von Efeu-Extrakten sind Atemwegserkrankungen, die häufig bei Kindern auftreten. Da Kinder oftmals ungern Arzneimittel zu sich nehmen, ist nunmehr eine erfolgreiche Behandlung mit weniger Verabreichungen möglich.

## Patentansprüche

1. Verfahren zur Herstellung eines Extrakts aus Efeublättern, welcher die Wirkstoffe Hederacosid C und α-Hederin aufweist, mit den Schritten:
a) Bereitstellen eines ersten α-Hederin-reichen Extraktes, der mindestens 3 % α-Hederin aufweist;
b) Bereitstellen eines zweiten Hederacosid C-reichen Extraktes, der mindestens 5 % Hederacosid C aufweist; und
c) Vermengen des ersten und des zweiten Extrakts zu einem Extrakt, der einen eingestellten Hederacosid C-Gehalt und einen eingestellten α-Hederin-Gehalt aufweist.

2. Verfahren nach Anspruch 1, **dadurch gekennzeichnet, dass** der α-Hederin-reiche Extrakt einen α-Hederin-Gehalt von mindestens 5 % aufweist.

3. Verfahren nach Anspruch 1 oder 2, **dadurch gekennzeichnet, dass** der Hederacosid C-reiche Extrakt einen Hederacosid C-Gehalt von mindestens 10 % und einen α-Hederin-Gehalt weniger als 2 % aufweist.

4. Verfahren nach einem der Ansprüche 1 bis 3, **dadurch gekennzeichnet, dass** der in Schritt c) zu gewinnende Extrakt einen eingestellten Hederacosid C-Gehalt von ca. 6,5 % und einen eingestellten α-Hederin-Gehalt von ca. 4,0 % aufweist.

5. Extrakt, hergestellt gemäß einem Verfahren nach einem der Ansprüche 1 bis 4, **dadurch gekennzeichnet, dass** er einen Hederacosid C-Gehalt von mindestens 5 % und einen α-Hederin-Gehalt von mindestens 3 % aufweist, mit der Ausnahme eines Extraktes, der 5,88 % Hederacosid C und 8,38 % α-Hederin enthält.

6. Extrakt nach Anspruch 5, **dadurch gekennzeichnet, dass** der Extrakt einen Hederacosid C-Gehalt von 6,5 % und einen α-Hederin-Gehalt von 4,0 % aufweist.

7. Verwendung eines Extraktes nach Anspruch 5 oder 6 zur Herstellung eines Arzneimittels.

8. Verwendung nach Anspruch 7, **dadurch gekennzeichnet, dass** das Arzneimittel zur Behandlung von Atemwegserkrankungen eingesetzt wird.

9. Arzneimittel, welches einen Extrakt nach einem der Ansprüche 5 oder 6 aufweist.

## Claims

1. Method for preparing an extract from ivy leaves which comprises as active ingredients hederacoside C and α-hederin, with the steps of
a) providing a first, α-hederin-rich extract having at least 3 % α-hederin;
b) providing a second, hederacoside C-rich extract having at least 5 % hederacoside C; and
c) blending said first and said second extract to give an extract which has an adjusted hederacoside C content and an adjusted α-hederin content.

2. Method of claim 1, **characterized in that** the α-hederin-rich extract has an α-hederin content of at least 5 %.

3. Method of claims 1 or 2, **characterized in that** the hederacoside C-rich extract has a hederacoside C content of at least 10 % and an α-hederin content of less than 2 %.

4. Method of anyone of claims 1 through 3, **characterized in that** the extract to be obtained in step c) has an adjusted hederacoside C content of about 6.5 % and an adjusted α-hederin content of about 4 %.

5. Extract, prepared by a method according to anyone of claims 1 through 4, **characterized in that** it contains a hederacoside C content of at least 5 % and an α-hederin content of at least 3 %, with the exception of an extract containing 5.88 % hederacoside C and 8.38 % α-hederin.

6. Extract of claim 5, **characterized in that** the extract has a hederacoside C content of 6.5 % and an α-hederin content of 4 %.

7. Use of an extract of claims 5 or 6 for preparing a pharmaceutical.

8. Use of claim 7, **characterized in that** the pharmaceutical is employed for the treatment of respiratory disorders.

9. Pharmaceutical comprising an extract of claims 5 or 6.

## Revendications

1. Procédé de fabrication d'un extrait de feuilles de lierre, lequel contient les substances actives hédéracoside C et hédérine α, comportant les étapes suivantes :
a) mise à disposition d'un premier extrait riche en hédérine α contenant au moins 3 % d'hédérine α ;
b) mise à disposition d'un second extrait riche en hédéracoside C contenant au moins 5 % d'hédéracoside C ; et
c) mélange du premier et du second extrait de manière à former un extrait présentant une teneur ajustée en hédéracoside C et une teneur ajustée en hédérine α.

2. Procédé selon la revendication 1, **caractérisé en ce que** l'extrait riche en hédérine α présente une teneur en hédérine α d'au moins 5 %.

3. Procédé selon la revendication 1, **caractérisé en ce que** l'extrait riche en hédéracoside C présente une teneur en hédéracoside C d'au moins 10 % et une teneur en hédérine α inférieure à 2 %.

4. Procédé selon l'une quelconque des revendications 1 à 3, **caractérisé en ce que** l'extrait à obtenir lors de l'étape c) présente une teneur ajustée en hédéracoside C d'environ 6,5 % et une teneur ajustée en hédérine α d'environ 4,0 %.

5. Extrait fabriqué selon un procédé selon l'une quelconque des revendications 1 à 4, **caractérisé en ce qu'**il présente une teneur en hédéracoside C d'au moins 5 % et une teneur en hédérine α d'au moins 3 %, à l'exception d'un extrait, lequel contient 5,88 % en hédéracoside C et 8,38 % en hédérine α.

6. Extrait selon la revendication 5, **caractérisé en ce que** l'extrait présente une teneur en hédéracoside C de 6,5 % et une teneur en hédérine α de 4,0 %.

7. Utilisation d'un extrait selon la revendication 5 ou 6 pour préparer un médicament.

8. Utilisation selon la revendication 7, **caractérisée en ce que** le médicament est utilisé pour le traitement des maladies des voies respiratoires.

9. Médicament contenant un extrait selon l'une quelconque des revendications 5 ou 6.
